# EUROPEAN PATENT APPLICATION

(11) **EP 3 915 537 A1**
(43) Date of publication of application: **01.12.2021**
(21) Application number: 20177151.6
(22) Date of filing: 28.05.2020
(51) Int. Cl.: A61K 8/99, A61Q 19/08

(54) **BIFIDOBACTERIA FOR PREVENTING, REDUCING OR TREATING SKIN AGING**

(71) Applicant: DuPont Nutrition Biosciences ApS, 1411 Copenhagen K (DK)
(72) Inventor: ANGLENIUS, Heli, 48210 Kotka (FI); HUUSKONEN, Laura, Tiina, Maria, 48210 Kotka (FI); TIIHONEN, Kirsti, 48210 Kotka (FI)
(74) Representative: DuPont EMEA

(57) **Abstract**

This invention relates to the use of a bacterium of the species *Bifidobacterium animalis* or a mixture thereof, particularly, a bacterium of *the Bifidobacterium animalis* ssp. *lactis* for preventing, reducing or treating skin aging in a subject. More particularly, but not exclusively, the invention relates to the use of *the Bifidobacterium animalis* ssp. *lactis* strains Bi-07, B420 and Bl-04 for preventing, reducing or treating skin aging in a subject.

## Description

### FIELD OF THE INVENTION

This invention relates to new uses of a bacterium of the genus *Bifidobacterium,* particularly to new uses of a bacterium of the species *Bifidobacterium animalis* or a mixture thereof. This invention also relates to compositions, skin care compositions, food products, dietary supplements, nutritional supplements, or pharmaceutical acceptable compositions comprising said bacterium.

### BACKGROUND OF THE INVENTION

The human skin is the biggest organ of the human body and it is the barrier that separates the body from the outer environment. One of its most important roles is protecting the body from inside-out water loss and outside-in microorganism infection. The human skin consists of two main layers of cells, epidermis and dermis. Epidermis is the outermost layer of the skin and is mainly formed of terminally differentiated keratinocytes and lipids, living dividing keratinocytes located beneath the terminally differentiated ones. The main function of epidermis is to form permeability barrier against environmental challenges, such as UV radiation, heat, chemicals, pollution, and pathogens, such as bacteria, fungi, parasites, and viruses. It also protects the body from uncontrolled water evaporation from inside out, maintaining the hydration balance and skin metabolism. Epidermal tight junctions play an important role in skin barrier function being flexible and interacting with the other components in skin (Bäsler K, et al., The role of tight junctions in skin barrier function and dermal absorption; J. Control Release, 2016 Nov 28; 242:105-118).

In the dermis, the most abundant cell type, dermal fibroblasts, are responsible of generating the connective tissue by producing extracellular matrix (ECM). This extracellular matrix (ECM) is composed of two main classes of macromolecules: proteoglycans (PGs) and fibrous proteins; the most abundant fibrous proteins are type I collagen fibrils, elastins, laminins and fibronectins (Frantz C, et al., The extracellular matrix at a glance, J. Cell Sci. 2010; 123 (24):4195-4200). During aging the collagen fibrils become fragmented, fibroblasts produce less ECM proteins and more ECM degrading matrix metalloproteinases (MMPs), that leads to imbalance in the ECM (Cole MA, et al., Extracellular matrix regulation of fibroblast function: redefining our perspective on skin aging, J Cell Commun Signal. 2018;12(1):35-43).

Although aging of the skin begins from the time one is born, it shows obvious and visible signs of aging when one becomes older. Skin aging can be caused by intrinsic aging or extrinsic aging. The term skin exposome describes these external and internal factors, and their interactions, that contribute to the aging of the skin. External factors, such as sun radiation (ultraviolet radiation, visible light and infrared radiation), heat, air pollution, tobacco smoke, alcohol consumption, nutrition, and a number of less well-defined miscellaneous factors, such as stress, sleep deprivation, temperature, and utilization of some cosmetic products, can contribute to the skin aging. These factors do not only affect the epidermis of the skin, but also the dermis, contributing to the aging of skin at multiple levels. Also, different disease conditions can contribute to aging. For example, the functions of epidermis are known to be disturbed in skin diseases such as psoriasis and atopic dermatitis (Kirschner N, et al.; Tight junctions and differentiation-a chicken or the egg question; Exp. Dermatol., 2012 Mar; 21(3):171-5*),* as well as during aging (Parrish A. R., et al., The impact of aging on epithelial barriers; Tissue Barriers, 2017 Oct 2; 5(4). Skin aging is therefore a complex biological process influenced by a combination of endogenous or intrinsic and exogenous or extrinsic factors.

As a major structural protein of the extracellular matrix (ECM), type I collagen is secreted by fibroblasts, forming more than 90% of the dry weight of the dermis. It is produced as pro-collagen which is then processed to mature collagen. Despite morphological and pathophysiological differences, the intrinsic and extrinsic skin aging shares several molecular similarities: reactive oxygen species are formed, and the resulting signalling cascade induces production of matrix metalloproteinases MMP-1 (interstitial collagenase), MMP-3 (stromelysin 1), and MMP-9 (gelatinase b), which degrade the dermal extracellular matrix, including collagen, and prevents the expression of pro-collagen-1 as well as the expression of inhibitors of the MMPs. Accumulation of fragmented collagen fibrils then prevents neocollagenesis and accounts for the further degradation of the extracellular matrix by means of positive feedback regulation that increases ROS amounts. For this reason, aged skin has increased amounts of degraded collagen. Furthermore, reduced expression of the connective tissue growth factor (CTGF) and reduced transforming growth factor (TGF)-b/Smad signalling are probably responsible for the loss of type I pro-collagen expression in aged skin. In some instances, such as during sun exposure, collagen production is diminished, and collagen degradation is increased. Therefore, it is important to keep the right MMP/pro-collagen 1 balance, to counteract the effect of excessive degration of collagen. It is also important to keep this balance, for instance in wound healing, as remodelling of the collagen by MMPs is important in removing wound debris, facilitate epithelialization and preventing scarring.

In addition, to changes in the collagen and MMPs, following alterations are induced in aging: nuclear and mitochondrial DNA mutations, single-stranded breaks, and oxidized pyrimidine bases are induced; membrane protein carbonylation and lipid peroxidation increases; apoptosis of epidermal keratinocytes increases; release of proinflammatory cytokines is increased, mainly IL-1, IL-6, TNF-alpha by keratinocytes, and increase in dermal receptors for epidermal growth factor, TNF-alpha, platelet activation factor, prostaglandins and insulin; NF-kappa B transcription factor becomes active, and this induces production of inflammatory cytokines which attract neutrophils that also express matrix metalloproteinases (metalloproteinase-8) contributing to the ECM degradation; TGF-beta is decreased, and this leads to altered collagen production and increase in the elastin, which induces a change in the skin structure, that then leads to formation of coarse textures, deep wrinkes, telangiectasia (spider veins) and pigmentation. TGF-β has differential effects in different cell types; local and systemic immunosuppression is induced by activation of immunosuppressive molecules such as IL-4, IL-10, IL-1β, TNF-alpha, and PGE2, or by alteration in cellular morphology, function or quantity of Langerhans cells and T lymphocytes. Antigen presentation in UV exposed skin is also impaired mainly due to suppression of important molecules expression such as MHC class II, lymphocyte function associated antigen-3 (LFA-3), ICAM-1, ICAM-3, B7, CD1a and CD40.

In addition to these changes, skin damage induced by tobacco smoking and air pollutants activate the aryl hydrocarbon receptor (AhR) pathway, a ligand-dependent transcription factor that mediates toxicity induced by several environmental contaminants, which may play a role in already known premature skin-aging effects.

Furthermore, skin barrier function is important for the protection of the skin from the external contaminants, and thus, proper function of the skin barrier is important also in preventing aging. Skin barrier is multi-cellular, with a highly organized, layered structure that provides a critical defense mechanism. In addition to lipids in the epidermis, tight junctions in the epidermis have been shown to contribute to the overall skin barrier function. In aged human skin, there is a decrease in the expression of the tight junction components claudin-1 and occludin, and aberrant increase in claudin-6, and this reduces the tight junction barrier function. Thus, epidermal tight junction barrier of the skin is disrupted during aging, and with following insults there is decreased recovery of function, due to decrease in the homotypic cell-cell adhesion molecules or alterations in the cytokine production. Especially IL-1alpha production is stimulated upon injury and knocking out the receptor for this cytokine exacerbates barrier dysfunction and decreases recovery. It has been also shown that proper skin barrier function in aged mice protects from age-associated systemic inflammation, and cytokines can also affect the barrier. Depending on the cytokine, it can be proinflammatory in nature, such as IL-1, IL-6, IL-8, TNF-alpha, or anti-inflammatory, such as IL-10 or IL-4, and these can be secreted by various cell types, including cells in the skin.

Also, there are changes occurring in skin blood vessels, acute UV exposure to human skin is known to induce angiogenesis to form new blood vessels in human skin. However, the blood vessels are decreased in chronically UV-damaged human skin, and cutaneous vessel size decreases with age in both intrinsically aged and chronic photoaged skin, and the reason for this difference is currently unknown. The epidermis-derived vascular endothelial growth factor (VEGF) is a potent angiogenic factor, and it has been shown to be increased for instance by retinoic acid in aged skin. TSP-1 and TSP-2 are of particular interest in wound repair and tissue remodeling due to their anti-angiogenic activity. Thrombospondin-1 (TSP-1) is a large multimeric ECM protein and has been well characterized as a potent endogenous inhibitor of angiogenesis in epithelial tissues, including the skin. It is downregulated in acute UV exposure. In acute UV exposure, that is often used as aging inducing factor, increase in VEGF and decrease in thrombospondin- 1 (TSP- 1) causes angiogenesis in papillary dermis with increased migration of elastase- positive leucocytes, leading to dermal elastic fibre damage, and this contribtutes to aging of the skin. Furthermore, thromospondin-2 (TSP-2) can influence collagen fibrillogenesis without being an integral component of fibrils. It is induced in response to injury and modulate the healing of dermal wounds. In the uninjured aged skin, the amount of TSP-2 is increased, and it has been associated with impaired wound healing of aged skin. Infrared radiation has been shown to increase both VEGF and TSP-2 expression and it is one confounding factor in the inflammatory process caused by IR radiation. IR radiation has been shown to increase MMP-1 and decrease pro-collagen-1, and chronic IR radiation has been shown to promote aging and cause skin wrinkling.

Various growth factors and their signalling routes are also involved in the skin aging process. The FGF family members increase the proliferation and activation of fibroblasts by stimulating the accumulation of collagen as well as stimulating endothelial cell division. FGFs comprise a growing group of structurally related polypeptide mitogens, which includes 23 different members. In addition to the previously discovered acid FGF (aFGF, FGF-1) and basic FGF (bFGF, FGF-2), the family also includes int-2 (FGF-3), and proto-oncogenes (FGF-4), FGF-5, and FGF-6, as well as keratinocyte growth factor (KGF, FGF-7), androgen-induced growth factor (AIGF, FGF-8), GAF (FGF-9), and FGF-10, among others. The basic FGF (FGF-2) reduces and prevents lines and wrinkles through the activation of new skin cells and stimulates the proliferation mainly fibroblasts and keratinocytes. Significant improvements in wrinkles, texture, elasticity, and density of the skin have been demonstrated, when FGF-2 was protected from thermal induced degradation. KGF appears to act specifically on epithelial cells, and when high concentrations of three growth factors: transforming growth factor beta 1, epidermal growth factor, and KGF, was applied on study subjects, visible signs of aging, including fine lines and wrinkles, improved in eighteen of the twenty subjects studied.

Because skin health and beauty are considered one of the principal factors representing overall "well-being" and the perception of "health" in humans, several anti-aging strategies have been developed during the last years. There is, however, a continuous need for products and methods capable of preventing, reducing or treating skin aging, not only for therapeutic reasons, but also for non-therapeutic uses, for example for cosmetic or skin care uses.

### OBJECT OF THE INVENTION

It is an object of the present invention to provide means for preventing, reducing or treating skin aging in a subject. In particular, it is the object of the present invention to provide means to maintain metabolic activity/cell viability in the skin, to increase the metabolic activity/cell viability in the skin or slow down the reduction of metabolic activity and/or cell viability in the skin. Furthermore, it is the object of the present invention to provide means to increase or maintain production of type I pro-collagen or to slow down the reduction of type I pro-collagen in the skin. It is yet another object of the present invention to provide means to increase collagen production or to maintain collagen metabolism or to slow down the reduction of the collagen metabolism in the skin and to maintain total matrix metalloproteinase 1 (MMP-1)/pro-collagen balance.

### SUMMARY OF THE INVENTION

In one aspect, the invention concerns a non-therapeutic use of a bacterium of the species *Bifidobacterium animalis* or a mixture thereof for preventing, reducing or treating skin aging in a subject.

In another aspect, the invention concerns a bacterium of the species *Bifidobacterium animalis* or a mixture thereof for use in prevention, reduction or treatment of skin aging in a subject.

In a further aspect, the invention concerns a method of preventing, reducing or treating skin aging in a subject, comprising administering a bacterium of the species *Bifidobacterium animalis* or a mixture thereof to said subject.

In another aspect, the invention concerns a composition, a skin care composition, a dietary supplement, a nutritional supplement, a food product or a pharmaceutical acceptable composition comprising a bacterium of the species *Bifidobacterium animalis* or a mixture thereof.

In particular, the invention concerns a bacterium of the subspecies *Bifidobacterium animalis* ssp. *lactis.*

In particular, the invention concerns a bacterium of the *Bifidobacterium animalis* ssp. *lactis* strain 420 (B420).

In particular, the invention concerns a bacterium of the *Bifidobacterium animalis* ssp. *lactis* strain Bi-07.

In particular, the invention concerns a bacterium of the *Bifidobacterium animalis* ssp. *lactis* strain BI-04.

### BRIEF DESCRIPTION OF THE FIGURES

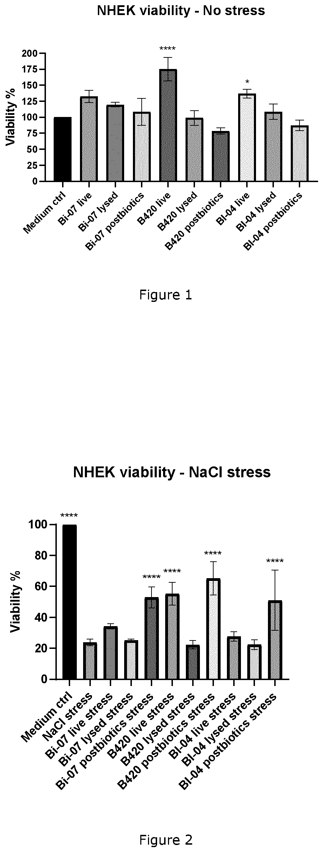

FIGURE 1 is a graph showing the effects of probiotics Bi-07, B420 and BI-04 on Normal Human Epidermal Keratinocyte (NHEK) cells metabolic activity (viability) compared to medium control values normalized to 100 percent metabolic activity (viability). Statistical significances shown as following: ****p<0.0001, *p<0.05. Ordinary one-way ANOVA, Dunnett's multiple comparisons test.
FIGURE 2 is a graph showing the effects of probiotics Bi-07, B420 and BI-04 on Normal Human Epidermal Keratinocyte (NHEK) cells metabolic activity (viability) under hyper-osmotic stress (induced by NaCl), compared to NaCl stress control, when medium control values are normalized to 100 percent metabolic activity (viability). Statistical significances shown as following: ****p<0.0001. Ordinary one-way ANOVA, Dunnett's multiple comparisons test.

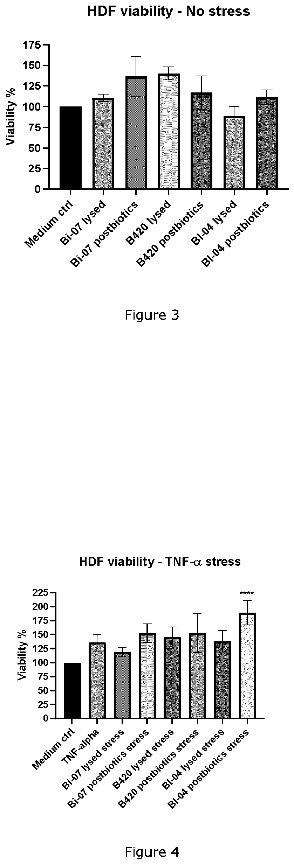

FIGURE 3 is a graph showing the effects of probiotics Bi-07, B420 and BI-04 on Human Dermal Fibroblast (HDF) cells metabolic activity (viability) compared to medium control values normalized to 100 percent metabolic activity (viability). Ordinary one-way ANOVA, Dunnett's multiple comparisons test.
FIGURE 4 is a graph showing the effects of probiotics Bi-07, B420 and BI-04 on Human Dermal Fibroblast (HDF) cells metabolic activity (viability) under inflammation stress (induced by TNF-a), compared to TNF-α stress control, when medium control values are normalized to 100 percent metabolic activity (viability). Statistical significances shown as following: ****p<0.0001. Ordinary one-way ANOVA, Dunnett's multiple comparisons test.

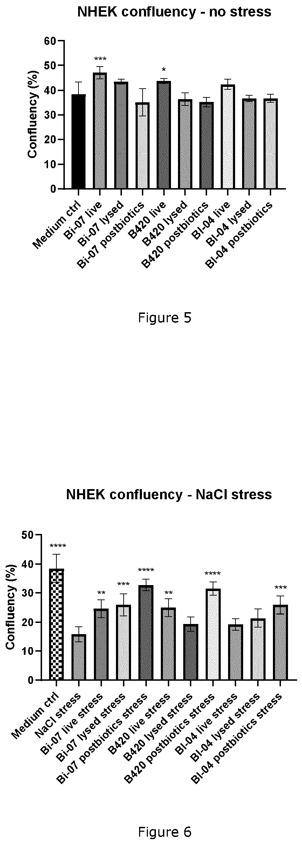

FIGURE 5 is a graph showing the effects of probiotics Bi-07, B420 and BI-04 on Normal Human Epidermal Keratinocyte (NHEK) cells confluency as percents compared to medium control. Statistical significances shown as following: ***p=0.0001, *p<0.05. Ordinary one-way ANOVA, Dunnett's multiple comparisons test.
FIGURE 6 is a graph showing the effects of probiotics Bi-07, B420 and BI-04 on Normal Human Epidermal Keratinocyte (NHEK) cells confluency under hyper-osmotic stress (induced by NaCl) as percents compared to NaCl stress control. Statistical significances shown as following: ****p<0.0001, ***p<0.001, **p<0.01. Ordinary one-way ANOVA, Dunnett's multiple comparisons test.

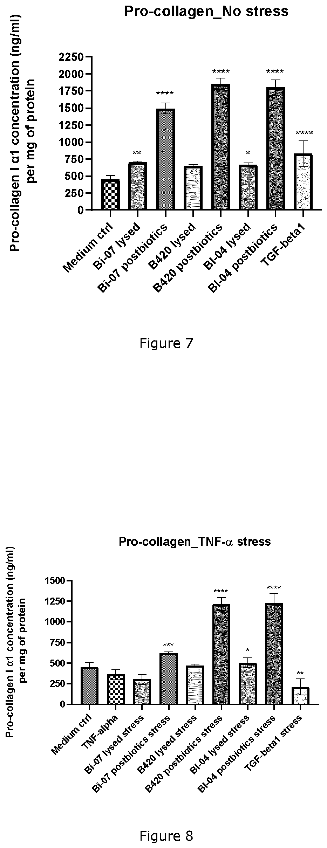

FIGURE 7 is a graph showing the effects of probiotics Bi-07, B420 and BI-04 on pro-collagen I α1 synthesis by Human Dermal Fibroblast (HDF) cells compared to medium control. The pro-collagen I α1 values are normalized with HDF cell lysate protein concentration and the results are presented as pro-collagen I α1 concentration (ng/ml) per mg of protein. Statistical significances shown as following: ****p<0.0001, **p<0.01, *p<0.05. Ordinary one-way ANOVA, Dunnett's multiple comparisons test.
FIGURE 8 is a graph showing the effects of probiotics Bi-07, B420 and BI-04 on pro-collagen I α1 synthesis by Human Dermal Fibroblast (HDF) cells under inflammation stress (induced by TNF-α) compared to TNF-α stress control. The pro-collagen I α1 values are normalized with HDF cell lysate protein concentration and the results are presented as pro-collagen I α1 concentration (ng/ml) per mg of protein. Statistical significances shown as following: ****p<0.0001, ***p<0.001, **p<0.01, *p<0.05. Ordinary one-way ANOVA, Dunnett's multiple comparisons test.

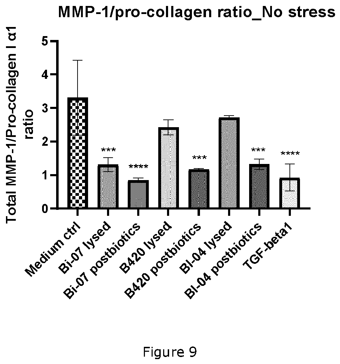

FIGURE 9 is a graph showing the effects of probiotics Bi-07, B420 and BI-04 on the ratio of produced total MMP-1 to synthesized pro-collagen I α1 by Human Dermal Fibroblast (HDF) cells compared to medium control. The total MMP-1 and pro-collagen I α1 values are normalized with HDF cell lysate protein concentration. Statistical significances shown as following: ****p<0.0001, ***p<0.001. Ordinary one-way ANOVA, Dunnett's multiple comparisons test.

### ADVANTAGES

It has surprisingly been found by the present inventors that the use of bacterium of the species *Bifidobacterium animalis* or a mixture thereof can maintain metabolic activity/cell viability in the skin, increase the metabolic activity/cell viability in the skin, or slow down reduction of metabolic activity and/or cell viability in the skin. It was further found out by the present inventors that bacterium of the species *Bifidobacterium animalis* or a mixture thereof can also increase or maintain production of type I pro-collagen, or slow down the reduction of type I pro-collagen in the skin, increase collagen production or maintain collagen metabolism or slow down the reduction the collagen metabolism in the skin and maintain total matrix metalloproteinase 1 (MMP-1)/pro-collagen balance. This shows the ability for bacterium of the species *Bifidobacterium animalis* or a mixture thereof to prevent, reduce or treat skin aging in a subject.

### DETAILED DISCLOSURE OF THE INVENTION

### Bacteria

The bacterium used in the present invention is selected from a bacterium of species *Bifidobacterium animalis* or a mixture thereof. Preferably the *Bifidobacterium* to be used in the present invention is a *Bifidobacterium* which is generally recognised as safe and, which is preferably GRAS approved. Generally recognized as safe (GRAS) is an American Food and Drug Administration (FDA) designation that a chemical or substance added to food is considered safe by experts, and so is exempted from the usual Federal Food, Drug, and Cosmetic Act (FFDCA) food additive tolerance requirements.

In one embodiment, the present invention relates to a non-therapeutic use of a bacterium of the species *Bifidobacterium animalis* or a mixture thereof for preventing, reducing or treating skin aging in a subject.

A non-therapeutic use includes, but it is not limited to, cosmetic or skin care uses or purposes. Therefore, the invention described herein also relates to a cosmetic or skin care use of a bacterium of the species *Bifidobacterium animalis* or a mixture thereof for preventing, reducing or treating skin aging in a subject.

In another embodiment, the present invention relates to a bacterium of the species *Bifidobacterium animalis* or a mixture thereof for use in prevention, reduction or treatment of skin aging in a subject.

In another embodiment, the present invention relates to a method of preventing, reducing or treating skin aging in a subject, said method comprising administering a bacterium of the species *Bifidobacterium animalis* or a mixture thereof to said subject.

The term "subject", as used herein, means an animal having a skin that separates and defines the animal inside from the outside. Preferably, the subject is a mammal, including for example livestock (including, but not limited to, cattle, horses, pigs, and sheep), and humans, or birds, such as chickens.

In some aspects of the present invention the animal is a companion animal (including pets), such as a dog or a cat for instance, but not limited to these, as it can be also a bird, such as parrot, or a turtle. In some aspects of the present invention, the subject may suitably be a human.

In one embodiment the subject is a human.

In one embodiment the subject may be female.

In one embodiment the subject may be male.

In one embodiment the subject may be with a non-binary gender.

In one embodiment the subject is not a child. The term "child" as used herein means a human 7 years of age or younger.

In one embodiment the subject is a human that is 8 years of age or older.

In one embodiment the subject is a human that is 16 years of age or older.

In one embodiment the subject is a human that is 18 years of age or older.

In one embodiment the subject is a healthy subject.

In one embodiment, the subject suffers from cancer, chronic inflammation, skin infection or any other transient or chronic medical condition responsible for skin aging.

In another embodiment of the present invention, the bacterium of the species *Bifidobacterium animalis* is a bacterium the subspecies *Bifidobacterium animalis* ssp. *lactis.*

In a further embodiment of the present invention, the bacterium of the species *Bifidobacterium animalis* or the mixture thereof maintains metabolic activity/cell viability in the skin, increases the metabolic activity/cell viability in the skin or slows down the reduction of metabolic activity and/or cell viability in the skin

Collagen is responsible for skin strength and structure; collagen homeostasis in the deeper part of the skin is maintained by balancing the degradative and synthetic pathways and the skewed balance between collagen production and degradation can lead to different skin ailments and aging. Therefore, in a further embodiment of the present invention, the bacterium of the species *Bifidobacterium animalis* or the mixture thereof increases or maintains production of type I pro-collagen or slows down the reduction of type I pro-collagen in the skin.

In another embodiment of the present invention, the bacterium of the species *Bifidobacterium animalis* or the mixture thereof, increases collagen production or maintains collagen metabolism, or slows down the reduction of the collagen metabolism in the skin.

In yet another embodiment of the present invention, the bacterium of the species *Bifidobacterium animalis* or the mixture thereof maintains the total matrix metalloproteinase 1 (MMP-1)/pro-collagen balance.

In a further embodiment, the prevention, reduction or treatment of the skin aging in a subject is attained by maintaining the metabolic activity/cell viability in the skin, increasing it or slowing down the reduction of metabolic activity and/or cell viability in the skin and/or by increasing or maintaining the production of type I pro-collagen or slowing down the reduction of type I pro-collagen in the skin and/or by increasing collagen production or maintaining collagen metabolism or slowing down the reduction of the collagen metabolism in the skin and/or by maintaining the total matrix metalloproteinase 1 (MMP-1)/pro-collagen balance.

In a further embodiment, the present invention relates to sking aging caused by intrinsic and extrinsic factors, such as, but not limited to, oxidative damage, DNA damage, impaired DNA repair, impaired cell division, excessive inflammation, immune diseases, excessive cell death, sun, sunburn, collagen damage, elastin damage, senescence, telomere shortening, impaired expression of antioxidant enzymes, impaired activity of antioxidant enzymes, infrared radiation, heat, hormonal reasons, poor nutrition, temperature, tobacco smoking, stress, sleep deprivation, pollution, or alcohol consumption.

Preferably, the *Bifidobacterium* used in the present invention is of the species *Bifidobacterium animalis.* More preferably, the *Bifidobacferium* used in the present invention is of the subspecies *Bifidobacterium animalis* ssp. *lactis.*

In a particularly preferred embodiment, the bacteria used in the present invention is *Bifidobacterium animalis* ssp. *lactis* strain 420 (B420). This strain is commercially available from DuPont Nutrition Biosciences ApS.

This strain of *Bifidobacterium animalis* ssp. *lactis* has also been deposited under the reference DGCC420 by DuPont Nutrition Biosciences ApS, of Langebrogade 1, DK-1411 Copenhagen K, Denmark, in accordance with the Budapest Treaty on 30 June 2015 at the Leibniz-Institut Deutsche Sammlung von Mikroorganismen und Zellkulturen GmbH (DSMZ), Inhoffenstrasse 7B, 38124 Braunschweig, Germany, where it is recorded under registration number DSM 32073.

In another particularly preferred embodiment, the bacteria used in the present invention are *Bifidobacterium animalis* ssp. *lactis* strain Bi-07 (DGCC12895). This strain is commercially available from DuPont Nutrition Biosciences ApS.

In another particularly preferred embodiment, the bacteria used in the present invention are *Bifidobacterium animalis* ssp. *lactis* strain BI-04 (DGCC2908). This strain is commercially available from DuPont Nutrition Biosciences ApS.

In the present invention, the *Bifidobacterium* used may be of the same type (species and strain) or may comprise a mixture of species and/or strains. It is therefore a particular embodiment of the present invention that the bacterium of the species *Bifidobacterium animalis* is selected from the group consisting of strain 420 (B420), strain Bi-07, strain BI-04 and any mixture thereof.

In one embodiment, the bacterium used in the present invention is a probiotic bacterium. In this specification the term "probiotic bacterium" is defined as covering any non-pathogenic bacterium which, when administered live, for example orally or topically, in adequate amounts, confer a health benefit on the host. Also, probiotic strains generally have an ability to survive the passage through the upper part of the digestive tract when intended for oral use. They are non-pathogenic, non-toxic and exercise their beneficial effect on health on the one hand via ecological interactions with the resident flora, and on the other hand via their ability to influence the immune system in a positive manner via the "MALT" (mucosa-associated lymphoid tissue). Probiotics can form part of the resident flora during the administration period. This colonization (or transient colonization) allows the probiotic bacteria to exercise a beneficial effect, such as normalization of perturbed microbiota by for instance through repression of potentially pathogenic bacteria, resisting their colonization, or competititive exclusion, and interactions with the immune system of the intestine or modulation of the fermentation of the gut microbiota. The probiotics mediate the health benefit also through production of enzymes or vitamins or other bioactives, through modulation of bile acid metabolism, and neutralization of harmful metabolites produced by other microbes.

In preferred embodiments, the bacterium used in the present invention is a probiotic *Bifidobacterium.*

The bacteria may be used in any form capable of exerting the effects described herein. For example, the bacteria may be viable, dormant, inactivated, lysed or dead bacteria. Preferably, the bacteria are in live form.

In a preferred embodiment, the bacteria are in a lysed form.

The bacteria may comprise whole bacteria or may comprise bacterial components. Examples of such components include, but not limited to, bacterial cell wall components such as peptidoglycan, lipoteichoic acid, bacterial nucleic acids such as DNA and RNA, bacterial membrane components, and bacterial structural components such as proteins, carbohydrates, lipids and combinations of these such as lipoproteins, glycolipids and glycoproteins.

The bacteria may also or alternatively comprise bacterial metabolites. In the present specification the term "bacterial metabolites" includes all molecules produced or modified by the bacteria as a result of bacterial metabolism during growth, survival, persistence, transit or existence of bacteria during the manufacture of the probiotic product and storage and during gastrointestinal transit in a mammal. Examples include, but not limited to, all organic acids, inorganic acids, bases, proteins and peptides, enzymes and co-enzymes, amino acids and nucleic acids, carbohydrates, lipids, glycoproteins, lipoproteins, glycolipids, vitamins, all bioactive compounds, metabolites containing an inorganic component, and all small molecules, for example nitrous molecules or molecules containing a sulphurous acid.

In another preferred embodiment, the bacteria are in a postbiotic form.

Postbiotics refers to products or metabolic byproducts, or metabolites, secreted by live bacteria generated through fermentation to the matrix, or released after bacterial lysis, such as, but not limited to, microbial cell fractions, proteins, enzymes, peptides, teichoic acids, peptidoglycan-derived muropeptides, polysaccharides, extracellular polysaccharides, cell surface proteins, pili-like structures, lipids and organic acids. These postbiotics because of their clear chemical structure, safety dose parameters, long shelf life and the content of various signaling molecules present anti-inflammatory, immunomodulatory, anti-obesogenic, antihypertensive, hypocholesterolemic, anti-proliferative, and/or antioxidant properties.

The invention further provides a mutant, a variant and/or a progeny of the bacterial strains B420, Bi-07 and BI-04.

As used herein, the term "mutant" refers to any microorganism resulting from modification of the strains B420, Bi-07 and BI-04. For example, a mutant may be a microorganism resulting from genetically modifying these strains.

As used herein, the term "variant" refers to a naturally occurring microorganism which is derived from the strains B420, Bi-07 and BI-04. For example, a variant may be a microorganism resulting from adaption to a particular environment or cell culture conditions.

As used herein, the term "progeny" means any microorganism resulting from the reproduction or multiplication of any one of the strains B420, Bi-07 or BI-04. Therefore, "progeny" means any direct descendant of any one of these strains. As such, the progeny strain may itself be identified as the same strain as the parent strain (*i.e.* strains B420, Bi-07 or BI-04). It will be apparent to one skilled in the art that due to the process of asexual reproduction, a progeny strain will be genetically virtually identical to the parent strain. Accordingly, in one embodiment, the progeny may be genetically identical to the parent strain and may be considered to be a "clone" of the parent strain. Alternatively, the progeny may be substantially genetically identical to the parent strain.

The mutant, variant or progeny may have at least 90, 95, 98, 99, 99.5 or 99.9% sequence identity over the entire length of the bacterial genome with their parent strain. Furthermore, the mutant, variant or progeny will retain the same phenotype as the deposited parent strain, for example the mutant, variant or progeny may demonstrate the same or equivalent effect on *in vitro* cell viability, cell confluency, collagen synthesis and metabolism.

For the purpose of the present invention, any mutant, variant and/or progeny of the bacterial strains B420, Bi-07 and BI-04 are considered to be the same as strains B420, Bi-07 and BI-04, respectively.

### Dosage

The *Bifidobacterium,* such as strains of *Bifidobacterium animalis* ssp. *lactis,* for example *Bifidobacterium animalis* ssp. *lactis* strain 420 (B420), strain Bi-07 and strain BI-04 used in accordance with the present invention may be present from 10⁶ to 10¹² CFU of bacteria/g of support, and more particularly from 10⁸ to 10¹² CFU of bacteria/g of support, preferably 10⁹ to 10¹² CFU/g of support.

Suitably, the *Bifidobacterium,* such as a strain of *Bifidobacterium animalis* ssp. *lactis,* for example *Bifidobacterium animalis* ssp. *lactis* strain 420 (B420), strain Bi-07 and strain BI-04, may be administered at a dosage of from about 10⁶ to about 10¹² CFU of microorganism/dose, preferably about 10⁸ to about 10¹² CFU of microorganism/dose. By the term "per dose" it is meant that this amount of microorganism is provided to a subject either per day or per intake, preferably per day. For example, if the microorganism is to be administered in a food product, for example in a yoghurt, then the yoghurt will preferably contain from about 10⁸ to 10¹² CFU of the microorganism. Alternatively, however, this amount of microorganism may be split into multiple administrations each consisting of a smaller amount of microbial loading - so long as the overall amount of microorganism received by the subject in any specific time, for instance each 24-hour period, is from about 10⁶ to about 10¹² CFU of microorganism, preferably 10⁸ to about 10¹² CFU of microorganism.

In accordance with the present invention an effective amount of at least one strain of a microorganism may be at least 10⁶ CFU of microorganism/dose, preferably from about 10⁶ to about 10¹² CFU of microorganism/dose, preferably about 10⁸ to about 10¹² CFU of microorganism/dose.

In one embodiment, preferably the *Bifidobacterium,* such as a strain of *Bifidobacterium animalis* ssp. *lactis,* for example *Bifidobacterium animalis* ssp. *lactis* strain 420 (B420), strain Bi-07 or strain BI-04 may be administered at a dosage of from about 10⁶ to about 10¹² CFU of microorganism/day, preferably about 10⁸ to about 10¹² CFU of microorganism/day. Hence, the effective amount in this embodiment may be from about 10⁶ to about 10¹² CFU of microorganism/day, preferably about 10⁸ to about 10¹² CFU of microorganism/day.

CFU stands for "colony-forming units". By "support" is meant a composition, a skin care composition, a dietary supplement, a nutritional supplement, a food product or a pharmaceutically acceptable composition.

In one embodiment, the present invention relates to bacteria of the species *Bifidobacterium animalis* or the mixture thereof, such as strains of *Bifidobacterium animalis* ssp. *lactis,* for example *Bifidobacterium animalis* ssp. *lactis* strain 420 (B420), strain Bi-07 and strain BI-04 as at least one of the components of a composition, a skin care composition, dietary supplements, nutritional supplements, food products or a pharmaceutical acceptable composition.

### Compositions

While is it possible to administer *Bifidobacferia* alone according to the present invention (*i.e.* without any support, diluent or excipient), the *Bifidobacteria* are typically and preferably administered on or in a support as part of a product, in particular as a component or at least as one of the components of a composition, a skin care composition, a dietary supplement, a nutritional supplement, a food product or a pharmaceutical acceptable composition. These products typically contain additional components well known to those skilled in the art.

In one embodiment, the present invention relates to a composition, a skin care composition or a pharmaceutical acceptable composition for oral administration or administration. A composition for topical application is a composition that is applied to a particular place on or in the body. Most often topical application or administration means application to body surfaces such as the skin or mucous membranes to treat certain ailments and, for example, to prevent, reduce or treat skin aging. The topical application can be achieved using a large range of products as support, such as, but not limited to, lotions, serums, jellies, creams, gels, emulsions, masks, patches, micellar water, sticks or ointments.

In another embodiment, the present invention relates to a composition, a skin care composition, a dietary supplement, a nutritional supplement a food product or pharmaceutical acceptable composition comprising a bacterium of the species *Bifidobacterium animalis* or a mixture thereof for oral administration.

A dietary supplement is any product intended to supplement the diet of a subject when taken orally as a pill, capsule, tablet, or liquid form.

### Skin care compositions

The present invention also relates to bacteria of the species *Bifidobacterium animalis* or a mixture thereof, such as strains of *Bifidobacterium animalis* ssp. *lactis,* for example *Bifidobacterium animalis* ssp. *lactis* strain 420 (B420), strain Bi-07 and strain BI-04 in the form of a skin care compositions or products including, but not limited to, aqueous solutions, emulsions, serums, jellies, masks, patches, face masks, peel-off masks, lotions, topical moisturizers, creams, pastes, balms, ointments, pomades, gels, liquids, sprays, foam and kits.

A skin care product, can also be in the form of makeup, lipstick, mascara, foundation, blush, eyeliner, lip gloss, macro emulsion, sunscreen, sun block, bath gel, shower gel, body wash, face wash, skin conditioner, cold cream, moisturizer, body spray, soap and body scrub.

A skin care composition or product may include a liquid lotion (true solution) comprising water as a solvent and water-soluble additives (solutes), such as but not limiting to an active, a fragrance, a color, a preservative, a pH adjuster, a chelating agent, or any one combination thereof.

A skin care product may also include a dispersion, for example an emulsion, such as, but not limited to: liquid in liquid (water in oil W/O, O/W, W/O/W), suspension (solid/liquid or liquid/solid), aerosol (liquid/gas or solid/gas), foam/mousse (gas/liquid or gas/emulsion, or gas/solid). An example of an Oil in Water (O/W) emulsion includes but is not limited to a combination of a water phase, an emulsifier, a fatty phase and an at least one additive. The water phase can comprise water, humectants and stabilizing agents, such as, but not limiting to, synthetic polymers, carbomers, natural polymers, xanthan gum, acacia gum, carrageenan, gellan, or any one combination thereof. Emulsifiers include, but are not limited to, anionic emulsifiers, cationic emulsifiers, non-ionic emulsifiers, amphoteric emulsifiers, silicone emulsifiers, autoemulsifying agents. Fatty phases (lipophilic ingredients) include, but are not limited to, waxes, butter, fatty esters, triglycerides, vegetal oil, mineral oil (parffinum), silicones, and thickeners/oil jellifying agents. Additives include, but are not limited to, preservative, fragrance (most often lipophilic), color, antioxidant, chelating agent, actives, pH adjuster (citric acid, lactic acid, AHA), neutralizers/strong basic agent like NaOH, Trimethylamine (for acrylic polymers to jellify) and powders.

A skin care product includes an aqueous gel comprising a water phase (including water, humectants, actives), a jellifying agent (such as but not limited to synthetic polymers, natural polymers, xanthan gum, acacia gum, carrageenan, gellan) and an additive (such as but not limited to fragrance, high HLB surfactant, color, actives, preservative system, pH adjuster, neutralizing agent, powders).

A skin care product includes a cleansing / surfactant system (such as but not limited to a shampoo, shower gel, micellar water) comprising a water phase (water, humectants), a surfactant, an additive (such as but not limited to fragrance, high HLB surfactant, color, actives, preservative system, pH adjuster, neutralizing agent, powders) and optionally a jellifying agent (such as but not limited to synthetic polymers, natural polymers, xanthan gum, acacia gum, carrageenan, gellan).

In one embodiment, a skin care product or formulation comprising an effective amount of a skin care composition, further comprises a bacterium of the species *Bifidobacterium animalis* or a mixture thereof.

In another embodiment, the skin product or formulation comprising an effective amount of a skin care composition, further comprises a bacterium of the species *Bifidobacterium animalis* selected from the group consisting of strain 420 (B420), strain Bi-07 and strain BI-04 and any mixture thereof.

The skin product or formulation comprising a bacterium of the species *Bifidobacterium animalis* provides a skin care benefit by preventing, reducing or treating skin aging by maintaining the metabolic activity/cell viability in the skin, increasing it, or slowing down the reduction of metabolic activity and/or cell viability in the skin; increasing or maintaining the production of type I pro-collagen or slowing down the reduction of type I pro-collagen in the skin or increasing collagen production or maintaining collagen metabolism, or slowing down the reduction of the collagen metabolism in the skin.

The bacterium of the species *Bifidobacterium animalis* ssp. *lactis,* for example *Bifidobacterium animalis* ssp. *lactis* strain 420 (B420), strain Bi-07 and strain BI-04 or a mixture thereof, used in accordance with the present invention, may be present from 0.1% to 10 % on a weight basis relative to a total weight of said skin care product. In one aspect, the preferable effective amount of the skin care composition described herein can be at least about 0.1% to 5% on a weight basis relative to a total weight of said skin care product. In one aspect, the effective amount of the skin care composition described herein can be at least about 0.1% to 10% on a weight basis relative to a total weight of said skin care product. The dermatologically acceptable component can be a dermatologically acceptable carrier comprising about 10% to about 99% on a weight basis relative to a total weight of the skin care product. In one aspect at least about 0.1 %, 1%, 2%, 3%, 4%, 5%, 6%, 7%, 8%, 9% up to 10% is the live bacteria, non-viable bacteria, bacterial lysate or postbiotic described herein on a weight basis relative to a total weight of said skin care product. The effective amount of the skin care composition in said skin care product can be at least about 0.1% to 5%, at least about 0.1% to 6%, at least about 0.1% to 7%, at least about 0.1% to 8%, at least about 0.1% to 9%, at least about 0.1% to 10% on a weight basis relative to a total weight of said formulation or skin care product. In one aspect the effective amount of the skin care composition in said skin care product can be at least about 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, 5.9%, 6.0%, 6.1%, 6.2%, 6.3%, 6.4%, 6.5%, 6.6%, 6.7%, 6.8%, 6.9%, 7.0%, 7.1%, 7.2%, 7.3%, 7.4%, 7.5%, 7.6%, 7.7%, 7.8%, 7.9%, 8.0%, 8.1%, 8.2%, 8.3%, 8.4%, 8.5%, 8.6%, 8.7%, 8.8%, 8.9%, 9.0%, 9.1%, 9.2%, 9.3%, 9.4%, 9.5%, 9.6%, 9.7%, 9.8%, 9.9%, to 10% on a weight basis relative to a total weight of said formulation or skin care product.

The skin care products described herein may further comprise one or more dermatologically or cosmetically acceptable components known or otherwise effective for use skin care, provided that the optional components are physically and chemically compatible with the essential components described herein, or do not otherwise unduly impair product stability, aesthetics, or performance. Non-limiting examples of such optional components are disclosed in International Cosmetic Ingredient Dictionary, Ninth Edition, 2002, and CTFA Cosmetic Ingredient Handbook, Tenth Edition, 2004.

In one aspect, the dermatologically or cosmetically acceptable component is a dermatologically acceptable carrier comprising from about 10 wt.% to about 99.9 wt.%, alternatively from about 50 wt.% to about 95 wt.%, and alternatively from about 75 wt.% to about 95 wt.%, of a dermatologically acceptable carrier. Carriers suitable for use with the composition(s) may include, for example, those used in the formulation of mousses, tonics, gels, skin moisturizers and lotions. The carrier may comprise water; organic oils; silicones such as volatile silicones, amino or non-amino silicone gums or oils, and mixtures thereof; mineral oils; plant oils such as olive oil, castor oil, rapeseed oil, coconut oil, wheatgerm oil, sweet almond oil, avocado oil, macadamia oil, apricot oil, safflower oil, candlenut oil, false flax oil, tamanu oil, lemon oil and mixtures thereof; waxes; and organic compounds such as C2-C10 alkanes, acetone, methyl ethyl ketone, volatile organic C1-C12 alcohols, esters of C1-C20 acids and of C1-C8 alcohols such as methyl acetate, butyl acetate, ethyl acetate, and isopropyl myristate, dimethoxyethane, diethoxyethane, C10-C30 fatty alcohols such as lauryl alcohol, cetyl alcohol, stearyl alcohol, and behenyl alcohol; C10-C30 fatty acids such as lauric acid and stearic acid; C10-C30 fatty amides such as lauric diethanolamide; C10-C30 fatty alkyl esters such as C10-C30 fatty alkyl benzoates; hydroxypropylcellulose, and mixtures thereof. In one aspect, the carrier comprises water, fatty alcohols, volatile organic alcohols, and mixtures thereof. Other carriers can be formulated by those of ordinary skill in the art.

The skin care products described herein may further comprise from about 0.1% to about 10%, and alternatively from about 0.2% to about 5.0%, of a gelling agent to help provide the desired viscosity to the composition(s). Non-limiting examples of suitable optional gelling agents include crosslinked carboxylic acid polymers; unneutralized crosslinked carboxylic acid polymers; unneutralized modified crosslinked carboxylic acid polymers; crosslinked ethylene/maleic anhydride copolymers; unneutralized crosslinked ethylene/maleic anhydride copolymers (e.g., EMA 81 commercially available from Monsanto); unneutralized crosslinked alkyl ether/acrylate copolymers (e.g., SALCARE™ SC90 commercially available from Allied Colloids); unneutralized crosslinked copolymers of sodium polyacrylate, mineral oil, and PEG-1 trideceth-6 (e.g., SALCARE™ SC91 commercially available from Allied Colloids); unneutralized crosslinked copolymers of methyl vinyl ether and maleic anhydride (e.g., STABILEZE™ QM-PVM/MA copolymer commercially available from International Specialty Products); hydrophobically modified nonionic cellulose polymers; hydrophobically modified ethoxylate urethane polymers (e.g., UCARE™ Polyphobe Series of alkali swellable polymers commercially available from Union Carbide); and combinations thereof. In this context, the term "unneutralized" means that the optional polymer and copolymer gelling agent materials contain unneutralized acid monomers.

The cosmetically acceptable medium may contain a fatty substance in a proportion generally of from about 10 to about 90% by weight relative to the total weight of the product, where the fatty phase containing at least one liquid, solid or semi-solid fatty substance. The fatty substance includes, but is not limited to, oils, waxes, gums, and so-called pasty fatty substances. Alternatively, the products may be in the form of a stable dispersion such as a water-in-oil or oil-in-water emulsion. Additionally, the skin care products may contain one or more conventional cosmetic or dermatological additives or adjuvants, including but not limited to, antioxidants, preserving agents, fillers, surfactants, UVA and/or UVB sunscreens, fragrances, thickeners, wetting agents and anionic, nonionic or amphoteric polymers, and dyes or pigments (colorant agents).

The dermatologically acceptable carrier may be a moisturizer formulation containing at least one emulsifier, at least one surfactant, or any combination thereof.

Skin care products can further comprise skin care active ingredient materials including sun screen agents, moisturizers, humectants, benefiting agents skin, depositing agents such as surfactants, occlusive agents, moisture barriers, lubricants, emollients, anti-aging agents, antistatic agents, abrasive, antimicrobials, conditioners, exfoliants, fragrances, viscosifying agents, salts, lipids, phospholipids, vitamins, foam stabilizers, pH modifiers, preservatives, suspending agents, silicone oils, silicone derivatives, essential oils, oils, fats, fatty acids, fatty acid esters, fatty alcohols, waxes, polyols, hydrocarbons, and mixtures thereof.

Other ingredients that may be included in a skin care product include, without limitation, at least one active ingredient for the treatment or prevention of skin ailments, providing a cosmetic effect, or for providing a moisturizing benefit to skin, such as zinc oxide, petrolatum, white petrolatum, mineral oil, cod liver oil, lanolin, dimethicone, hard fat, vitamin A, allantoin, calamine, kaolin, glycerin, or colloidal oatmeal, and combinations of these, one or more natural moisturizing factors (such as ceramides, hyaluronic acid, glycerin, squalane, amino acids, cholesterol, fatty acids, triglycerides, phospholipids, glycosphingolipids, urea, linoleic acid, glycosaminoglycans, mucopolysaccharide, sodium lactate, or sodium pyrrolidone carboxylate, for example), glycerides, apricot kernel oil, canola oil, squalane, squalene, coconut oil, corn oil, jojoba oil, jojoba wax, lecithin, olive oil, safflower oil, sesame oil, shea butter, soybean oil, sweet almond oil, sunflower oil, tea tree oil, shea butter, palm oil, cholesterol, cholesterol esters, wax esters, fatty acids, and orange oil.

### Food Product

In one embodiment, the *Bifidobacteria* are employed according to the invention in a food product, such as a food supplement, a drink or a powder based on milk. Here, the term "food" is used in a broad sense and covers food for humans as well as food for animals (*i.e.* a feed). In a preferred aspect, the food is for human consumption.
The food may be in the form of a solution or as a solid, depending on the use and/or the mode of application and/or the mode of administration.

When used as, or in the preparation of, a food, such as functional food, the bacteria of the present invention may be used in conjunction with one or more of: a nutritionally acceptable carrier, a nutritionally acceptable diluent, a nutritionally acceptable excipient, a nutritionally acceptable adjuvant, a nutritionally active ingredient.

By way of example, the bacteria of the present invention can be used as an ingredient to soft drinks, a fruit juice or a beverage comprising whey protein, health teas, cocoa drinks, milk drinks and lactic acid bacteria drinks, yoghurt and drinking yoghurt, cheese, ice cream, water ices and desserts, confectionery, biscuits cakes and cake mixes, snack foods, balanced foods and drinks, fruit fillings, care glaze, chocolate bakery filling, cheese cake flavoured filling, fruit flavoured cake filling, cake and doughnut icing, instant bakery filling creams, fillings for cookies, ready-to-use bakery filling, reduced calorie filling, adult nutritional beverage, vegetable milk, acidified soy/juice beverage, aseptic/retorted chocolate drink, bar mixes, beverage powders, calcium fortified soy/plain and chocolate milk, calcium fortified coffee beverage.

Advantageously, where the product is a food product, the *Bifidobacteria* should remain effective through the normal "sell-by" or "expiration" date during which the food product is offered for sale by the retailer. Preferably, the effective time should extend past such dates until the end of the normal freshness period when food spoilage becomes apparent. The desired lengths of time and normal shelf life will vary from foodstuff to foodstuff and those of ordinary skill in the art will recognise that shelf-life times will vary upon the type of foodstuff, the size of the foodstuff, storage temperatures, processing conditions, packaging material and packaging equipment.

### Medical Food

In one embodiment, the bacterium of the present invention is in the form of a medical food product.

Preferably, the *Bifidobacterium* of the present invention, such as strains of *Bifidobacterium animalis* ssp. *lactis,* for example *Bifidobacterium animalis* ssp. *lactis* strain 420 (B420), strain Bi-07 or strain BI-04 are in the form of a medical food product.

By "medical food" it is meant a food product which is formulated to be consumed or administered with or without the supervision of a physician and which is intended for a specific skin condition or other medical condition for which distinctive nutritional requirements, based on recognized scientific principles, are established.

### Pharmaceutical composition

The bacteria of the present invention may be used as - or in the preparation of - a pharmaceutical composition. Here, the term "pharmaceutical" is used in a broad sense - and covers pharmaceuticals for humans as well as pharmaceuticals for animals (i.e. veterinary applications).

In a preferred embodiment, the pharmaceutical acceptable composition is a medicament.

The pharmaceutical composition can be for therapeutic purposes - which may be curative or palliative or preventative in nature. The pharmaceutical composition may even be for diagnostic purposes.

In a preferred embodiment of the present invention, the medicament is for topical application.

In another preferred embodiment of the present invention, the medicament is for oral administration.

A pharmaceutically acceptable composition or support may be for example a formulation or support in the form of creams, foams, gels, lotions, and ointments of compressed tablets, tablets, capsules, ointments, suppositories or drinkable solutions. Other suitable forms are provided below.

When used as - or in the preparation of - a pharmaceutical, the composition of the present invention may be used in conjunction with one or more of: a pharmaceutically acceptable carrier, a pharmaceutically acceptable diluent, a pharmaceutically acceptable excipient, a pharmaceutically acceptable adjuvant, a pharmaceutically active ingredient.

The pharmaceutical may be in the form of a solution or as a solid - depending on the use and/or the mode of application and/or the mode of administration.

The *Bifidobacteria* of the present invention may be used as pharmaceutical ingredients. Here, the composition may be the sole active component, or it may be at least one of a number (*i.e.* 2 or more) of active components.

The pharmaceutical ingredient may be in the form of a solution or as a solid - depending on the use and/or the mode of application and/or the mode of administration.

The bifidobacteria may be used according to the present invention in any suitable form - whether when alone or when present in a combination with other components or ingredients. Likewise, combinations comprising the bacteria of the present invention and other components and/or ingredients (*i.e.* ingredients - such as food ingredients, functional food ingredients or pharmaceutical ingredients) may be used in any suitable form.

The bifidobacteria may be used according to the present invention in the form of solid or liquid preparations or alternatives thereof. Examples of solid preparations include, but are not limited to tablets, capsules, dusts, granules and powders which may be wettable, spray-dried or freeze-dried. Examples of liquid preparations include, but are not limited to, aqueous, organic or aqueous-organic solutions, suspensions and emulsions.

Suitable examples of forms include one or more of: tablets, pills, capsules, ovules, solutions or suspensions, which may contain flavouring or colouring agents, for immediate-, delayed-, modified-, sustained-, pulsed- or controlled-release applications.

By way of example, if the bacteria of the present invention is used in a tablet form - such for use as a functional ingredient - the tablets may also contain one or more of: excipients such as microcrystalline cellulose, lactose, sodium citrate, calcium carbonate, dibasic calcium phosphate and glycine; disintegrants such as starch (preferably corn, potato or tapioca starch), sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included.

Examples of nutritionally acceptable carriers for use in preparing the forms include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethrai fatty acid esters, hydroxymethylcellulose, polyvinylpyrrolidone, and the like.

Preferred excipients for the forms include lactose, starch, a cellulose, milk sugar or high molecular weight polyethylene glycols.

For aqueous suspensions and/or elixirs, the bacteria of the present invention may be combined with various sweetening or flavouring agents, colouring matter or dyes, with emulsifying and/or suspending agents and with diluents such as water, propylene glycol and glycerin, and combinations thereof.

The forms may also include gelatin capsules; fibre capsules, fibre tablets etc.; or even fibre beverages.

Further examples of form include creams. For some aspects the microorganism used in the present invention may be used in pharmaceutical and/or cosmetic creams such as sun creams and/or after-sun creams for example.

In one aspect, the bacteria according to the present invention may be administered in an aerosol, for example by way of a nasal spray, for instance for administration to the respiratory tract.

### Prebiotics

In one embodiment, the use of the bacterium of the present invention may further comprise the use of one or more fibres and/or prebiotics.

Prebiotics are defined as a substrate that is selectively utilized by host microorganisms conferring a health benefit. These are generally ingredients that beneficially affect the health of the host by selectively stimulating the growth and/or activity of one or a limited number of bacteria (particularly, although not limited to bifidobacteria and/or lactic acid bacteria), and thus improve host health. The prebiotic can be applied to oral route, but it can be also applied to other microbioally colonized sites, for instance on the skin topically. Typically, prebiotics are carbohydrates (such as oligosaccharides), but the definition does not preclude non-carbohydrates, such as polyphenols, or polyunsaturated fatty acids or other ingredients that can be utilized selectively by a limited number of bacteria to confer a health benefit. The most prevalent forms of prebiotics are nutritionally classed as soluble fibres. To some extent, many forms of dietary fibres exhibit some level of prebiotic effect.

In one embodiment, a prebiotic is a selectively fermented ingredient that allows specific changes, both in the composition and/or activity in the gastrointestinal or skin microflora that confers benefits upon host well-being and health.

Suitably, the prebiotic may be used according to the present invention in an amount of 0.01 to 100 g/day, preferably 0.1 to 50 g/day, more preferably 0.5 to 20 g/day. In one embodiment, the prebiotic may be used according to the present invention in an amount of 1 to 10 g/day, preferably 2 to 9 g/day, more preferably 3 to 8 g/day. In another embodiment, the prebiotic may be used according to the present invention in an amount of 5 to 50 g/day, preferably 10 to 25 g/day.

Examples of dietary sources of prebiotics include soybeans, inulin sources (such as Jerusalem artichoke, jicama, and chicory root), raw oats, unrefined wheat, unrefined barley and yacon.

Examples of suitable prebiotics include alginate, xanthan, pectin, locust bean gum (LBG), inulin, guar gum, galacto-oligosaccharide (GOS), fructo-oligosaccharide (FOS), polydextrose (*i.e.* Litesse®), lactitol, L-Arabinose, D-Xylose, L-Rhamnose, D-Mannose, L-Fucose, inositol, sorbitol, mannitol, xylitol, fructose, carrageenan, alginate, microcrystalline cellulose (MCC), betaine, lactosucrose, soybean oligosaccharides, isomaltulose (Palatinose TM), isomalto-oligosaccharides, gluco-oligosaccharides, xylooligosaccharides, manno-oligosaccharides, beta-glucans, cellobiose, raffinose, gentiobiose, melibiose, xylobiose, cyciodextrins, isomaltose, trehalose, stachyose, panose, pullulan, verbascose, galactomannans, (human) milk oligosaccharides and all forms of resistant starches.

The combination of bifidobacterium and one or more fibres and/or prebiotics according to the present invention exhibits a synergistic effect in certain applications (*i.e.* an effect which is greater than the additive effect of the bacteria when used separately).

In one embodiment, the bacterium of the species *Bifidobacterium animalis* or a mixture thereof is used in combination with one or more fibres and/or prebiotic.

In another embodiment, the bacterium of the species *Bifidobacterium animalis* or a mixture thereof used in combination with one or more fibres and/or prebiotic is of the subspecies *Bifidobacterium animalis* ssp. *lactis.*

In a particularly preferred embodiment, the *Bifidobacterium* or a mixture thereof used in combination with one or more fibres and/or prebiotic is the *Bifidobacterium animalis* ssp. *lactis* strain 420 (B420).

In a particularly preferred embodiment, the *Bifidobacterium* or a mixture thereof used in combination with one or more fibres and/or prebiotic is the *Bifidobacterium animalis* ssp. *lactis* strain Bi-07 (Bi-07).

In a particularly preferred embodiment, the *Bifidobacterium* or a mixture thereof used in combination with one or more fibres and/or prebiotic is the *Bifidobacterium animalis* ssp. *lactis* strain BI-04 (BI-04).

Suitably, the prebiotic used is polydextrose, lactitol, inositol, L-Arabinose, D-Xylose, L-Rhamnose, D-Mannose, L-Fucose, sorbitol, mannitol, xylitol, fructose, carrageenan, alginate, microcrystalline cellulose (MCC), milk oligosaccharide or betaine.

In a further aspect, the invention relates to a composition, a skin care composition, dietary supplements, nutritional supplements, food products or a pharmaceutical acceptable composition comprising a bacterium of the species *Bifidobacterium animalis* or a mixture thereof and one or more fibres and/or a prebiotic.

In one aspect, the prebiotic can be a metabolite produced by a bacterium of species *Bifidobacterium animalis* or mixture thereof, and this metabolite can be utilized as cross-feeding of other microbes, for instance when applied topically on the skin, affecting the health beneficially.

In one aspect, the prebiotic can be a metabolite produced by a bacterium of subspecies *Bifidobacterium animalis* spp. *lactis,* and this metabolite can be utilized as cross-feeding of other microbes, for instance when applied topically on the skin, affecting the health beneficially.

In one aspect, the prebiotic can be a metabolite produced by a bacterium of subspecies *Bifidobacterium animalis* spp. *lactis* 420 (B420).

In one aspect, the prebiotic can be a metabolite produced by a bacterium of subspecies *Bifidobacterium animalis* spp. *lactis* Bi-07.

In one aspect, the prebiotic can be a metabolite produced by a bacterium of subspecies *Bifidobacterium animalis* spp. *lactis* BI-04.

### Embodiments of the invention

For the avoidance of doubt, some of the embodiments the present invention relates to are set out below:
Embodiment 1: Non-therapeutic use of a bacterium of the species *Bifidobacterium animalis* or a mixture thereof for preventing, reducing or treating skin aging in a subject.
Embodiment 2: The non-therapeutic use according to embodiment 1, wherein the bacterium of the species *Bifidobacterium animalis* is of the subspecies *Bifidobacterium animalis* ssp. *lactis.*
Embodiment 3: The non-therapeutic use according to embodiments 1 or 2, wherein the bacterium of the species *Bifidobacterium animalis* or the mixture thereof maintains metabolic activity/ cell viability in the skin, increases the metabolic activity/ cell viability in the skin or slows down the reduction of metabolic activity and/or cell viability in the skin
Embodiment 4: The non-therapeutic use according to embodiments 1-3, wherein the bacterium of the species *Bifidobacterium animalis* or the mixture thereof increases or maintains production of type I pro-collagen or slows down the reduction of type I pro-collagen in the skin.
Embodiment 5: The non-therapeutic use according to embodiments 1-3, wherein the bacterium of the species *Bifidobacterium animalis* or the mixture thereof increases collagen production or maintains collagen metabolism or slows down the reduction of collagen metabolism in the skin
Embodiment 6: The non-therapeutic use according to embodiments 1-3, wherein the bacterium of the species *Bifidobacterium animalis* or the mixture thereof maintains the total matrix metalloproteinase 1 (MMP-1)/pro-collagen balance.
Embodiment 7: The non-therapeutic use according to any one of embodiments 1-6, wherein the skin aging is caused by oxidative damage, DNA damage, impaired DNA repair, impaired cell division, excessive inflammation, immune diseases, excessive cell death, sun, sunburn, collagen damage, elastin damage, senescence, telomere shortening, impaired expression of antioxidant enzymes or impaired activity of antioxidant enzymes, infrared radiation, ultra-violet radiation, heat, hormonal reasons, poor nutrition, temperature, tobacco smoking, stress, sleep deprivation, pollution, or alcohol consumption.
Embodiment 8: The non-therapeutic use according to any one of the embodiments 1-7, wherein the bacterium of the species *Bifidobacterium animalis* is, or the mixture thereof comprises, strain 420 (B420).
Embodiment 9: The non-therapeutic use according to any one of the embodiments 1-7, wherein the bacterium of the species *Bifidobacterium animalis* is, or the mixture thereof comprises, strain Bi-07.
Embodiment 10: The non-therapeutic use according to any one of the embodiments 1-7, wherein the bacterium of the species *Bifidobacterium animalis* is, or the mixture thereof comprises, strain BI-04.
Embodiment 11: The non-therapeutic use according to any one of the embodiments 1-7, wherein the bacterium of the species *Bifidobacterium animalis* is selected from the group consisting of strain 420 (B420), strain Bi-07, strain BI-04 and any mixture thereof.
Embodiment 12: The non-therapeutic use according to any one of the embodiments 1-11, wherein the bacterium of the species *Bifidobacterium animalis* or the mixture thereof are in a live form.
Embodiment 13: The non-therapeutic use according to any one of the embodiments 1-11, wherein the bacterium of the species *Bifidobacterium animalis* or the mixture thereof are in a lysed form.
Embodiment 14: The non-therapeutic use according to any one of the embodiments 1-11, wherein the bacterium of the species *Bifidobacterium animalis* or the mixture thereof are in a postbiotic form.
Embodiment 15: The non-therapeutic use according to any one of embodiments 1 to 14, further comprising the use of one or more fibers and/or prebiotics.
Embodiment 16: The non-therapeutic use according to any one of embodiments 1 to 15, wherein the bacterium of the species *Bifidobacterium animalis* or the mixture thereof is at least one component of a composition, a skin care composition, a dietary supplement, a nutritional supplement, a food product or a pharmaceutical acceptable composition.
Embodiment 17: The non-therapeutic use according to embodiment 16, wherein the composition, the skin care composition or the pharmaceutical acceptable composition are for topical application.
Embodiment 18: The non-therapeutic use according to embodiment 16, wherein the composition, the skin care composition, the dietary supplement, the nutritional supplement, the food product or the pharmaceutical acceptable composition are for oral administration.
Embodiment 19: The non-therapeutic use according to embodiment 16, wherein the food product is a medical food product.
Embodiment 20: The non-therapeutic use according to embodiment 16, wherein the pharmaceutical acceptable composition is a medicament.
Embodiment 21: The non-therapeutic use according to embodiment 16, wherein the medicament is for topical application.
Embodiment 22: Bacterium of the species *Bifidobacterium animalis* or a mixture thereof for use in prevention, reduction or treatment of skin aging in a subject.
Embodiment 23: The bacterium for use according to embodiment 22, wherein said bacterium of the species *Bifidobacterium animalis* is of the subspecies *Bifidobacterium animalis* ssp. *lactis.*
Embodiment 24: The bacterium for use according to embodiments 22 or 23, wherein the bacterium of the species *Bifidobacterium animalis* or the mixture thereof maintains metabolic activity/ cell viability in the skin, increases the metabolic activity/ cell viability in the skin or slows down reduction of metabolic activity and/or cell viability in the skin.
Embodiment 25: The bacterium for use according to embodiments 22-24, wherein the bacterium of the species *Bifidobacterium animalis* or the mixture thereof increases or maintains production of type I pro-collagen or slows down the reduction of type I pro-collagen in the skin.
Embodiment 26: The bacterium for use according to embodiments 22-24, wherein the bacterium of the species *Bifidobacterium animalis* or the mixture thereof increases collagen production or maintains collagen metabolism or slows down the reduction of collagen metabolism in the skin.
Embodiment 27: The bacterium for use according to embodiments 21-23, wherein the bacterium of the species *Bifidobacterium animalis* or the mixture thereof maintains the total matrix metalloproteinase 1 (MMP-1)/pro-collagen balance.
Embodiment 28: The bacterium for use according to any one of embodiments 22-27, wherein the skin aging is caused by oxidative damage, DNA damage, impaired DNA repair, impaired cell division, excessive inflammation, immune diseases, excessive cell death, sun, sunburn, collagen damage, elastin damage, senescence, telomere shortening, impaired expression of antioxidant enzymes or impaired activity of antioxidant enzymes, infrared radiation, ultra-violet radiation, heat, hormonal reasons, poor nutrition, temperature, tobacco smoking, stress, sleep deprivation, pollution, or alcohol consumption.
Embodiment 29: The bacterium for use according to any one of the embodiments 22-28, wherein the bacterium of the species *Bifidobacterium animalis* is, or the mixture thereof comprises, strain 420 (B420).
Embodiment 30: The bacterium for use according to any one of the embodiments 22-28, wherein the bacterium of the species *Bifidobacterium animalis* is, or the mixture thereof comprises, strain Bi-07.
Embodiment 31: The bacterium for use according to any one of the embodiments 22-28, wherein the bacterium of the species *Bifidobacterium animalis* is, or the mixture thereof comprises, strain BI-04.
Embodiment 32: The bacterium for use according to any one of the embodiments 22-28, wherein the bacterium of the species *Bifidobacterium animalis* is selected from the group consisting of strain 420 (B420), strain Bi-07, strain BI-04 and any mixture thereof.
Embodiment 33: The bacterium for use according to any one of the embodiments 22-32, wherein the bacterium of the species *Bifidobacterium animalis* or the mixture thereof are in a live form.
Embodiment 34: The bacterium for use according to any one of the embodiments 22-32, wherein the bacterium of the species *Bifidobacterium animalis* or the mixture thereof are in a lysed form.
Embodiment 35: The bacterium for use according to any one of the embodiments 22-32, wherein the bacterium of the species *Bifidobacterium animalis* or the mixture thereof are in a postbiotic form.
Embodiment 36: The bacterium for use according to any one of embodiments 22 to 35, further comprising the use of one or more fibers and/or prebiotics.
Embodiment 37: The bacterium for use according to any one of embodiments 22 to 36, wherein the bacterium of the species *Bifidobacterium animalis* or the mixture thereof is at least one component of a composition, a skin care composition, a dietary supplement, a nutritional supplement, a food product or a pharmaceutical acceptable composition.
Embodiment 38: The bacterium for use according to embodiment 37, wherein the composition, the skin care composition or the pharmaceutical acceptable composition are for topical application.
Embodiment 39: The bacterium for use according to embodiment 37, wherein the composition, the skin care composition, the dietary supplement, the nutritional supplement, the food product or the pharmaceutical acceptable composition are for oral administration.
Embodiment 40: The bacterium for use according to embodiment 37, wherein the food product is a medical food product.
Embodiment 41: The bacterium for use according to embodiment 37, wherein the pharmaceutical acceptable composition is a medicament.
Embodiment 42: The bacterium for use according to embodiment 37, wherein the medicament is for topical application.
Embodiment 43: Method of preventing, reducing or treating skin aging in a subject, said method comprising administering a bacterium of the species *Bifidobacterium animalis* or a mixture thereof to said subject.
Embodiment 44: The method according to embodiment 43, wherein the bacterium of the species *Bifidobacterium animalis* is of the subspecies *Bifidobacterium animalis* ssp. *lactis.*
Embodiment 45: The method according to embodiment 43 or 44, wherein the bacterium of the species *Bifidobacterium animalis* or the mixture thereof maintains metabolic activity/ cell viability in the skin, increases the metabolic activity/ cell viability in the skin or slows down the reduction of metabolic activity and/or cell viability in the skin.
Embodiment 46: The method according to embodiments 43-45, wherein the bacterium of the species *Bifidobacterium animalis* or the mixture thereof increases or maintains production of type I pro-collagen or slows down the reduction of type I pro-collagen in the skin.
Embodiment 47: The method according to embodiments 43-45, wherein the bacterium of the species *Bifidobacterium animalis* or the mixture thereof increases collagen production or maintains collagen metabolism or slows down the reduction of the collagen metabolism in the skin
Embodiment 48: The method according to embodiments 43-45, wherein the bacterium of the species *Bifidobacterium animalis* or the mixture thereof maintains total matrix metalloproteinase 1 (MMP-1)/pro-collagen balance.
Embodiment 49: The method according to any one of embodiments 43-48, wherein the skin aging is caused by oxidative damage, DNA damage, impaired DNA repair, impaired cell division, excessive inflammation, immune diseases, excessive cell death, sun, sunburn, collagen damage, elastin damage, senescence, telomere shortening, impaired expression of antioxidant enzymes or impaired activity of antioxidant enzymes, infrared radiation, ultra-violet radiation, heat, hormonal reasons, poor nutrition, temperature, tobacco smoking, stress, sleep deprivation, pollution, or alcohol consumption.
Embodiment 50: The method according to any one of the embodiments 43-49, wherein the bacterium of the species *Bifidobacterium animalis* is, or the mixture thereof comprises, strain 420 (B420).
Embodiment 51: The method according to any one of the embodiments 43-49, wherein the bacterium of the species *Bifidobacterium animalis* is, or the mixture thereof comprises, strain Bi-07.
Embodiment 52: The method according to any one of the embodiments 43-49, wherein the bacterium of the species *Bifidobacterium animalis* is, or the mixture thereof comprises, strain BI-04.
Embodiment 53: The method according to any one of the embodiments 43-49, wherein the bacterium of the species *Bifidobacterium animalis* is selected from the group consisting of strain 420 (B420), strain Bi-07, strain BI-04 and any mixture thereof.
Embodiment 54: The method according to any one of the embodiments 43-53, wherein the bacterium of the species *Bifidobacterium animalis* or the mixture thereof are in a live form.
Embodiment 55: The method according to any one of the embodiments 43-53, wherein the bacterium of the species *Bifidobacterium animalis* or the mixture thereof are in a lysed form.
Embodiment 56: The method according to any one of the embodiments 43-53, wherein the bacterium of the species *Bifidobacterium animalis* or the mixture thereof are in a postbiotic form.
Embodiment 57: The method according to any one of embodiments 43 to 56, further comprising the use of one or more fibers and/or prebiotics.
Embodiment 58: The method according to any one of embodiments 43 to 57, wherein the bacterium of the species *Bifidobacterium animalis* or the mixture thereof is at least one component of a composition, a skin care composition, a dietary supplement, a nutritional supplement, a food product or a pharmaceutical acceptable composition.
Embodiment 59: The method according to embodiment 58, wherein the composition, the skin care composition or the pharmaceutical acceptable composition are for topical application.
Embodiment 60: The method according to embodiment 58, wherein the composition, the skin care composition, the dietary supplement, the nutritional supplement, the food product or the pharmaceutical acceptable composition are for oral administration.
Embodiment 61: The method according to embodiment 58, wherein the food product is a medical food product.
Embodiment 62: The method according to embodiment 58, wherein the pharmaceutical acceptable composition is a medicament.
Embodiment 63: The method according to embodiment 58, wherein the medicament is for topical application.

### Examples

### Materials and methods

Three DuPont probiotic strains, *Bifodobacterium animalis* spp. *lactis* B420, Bi-07 and BI-04, were tested in *in vitro* with Normal Human Epidermal Keratinocyte (NHEK) cells and Human Dermal Fibroblast (HDF) cells for their effects on skin hydration, moisturization and anti-aging properties. The probiotic strains were tested as live, lysed or as a postbiotic solution with NHEK cells with and without hyperosmotic stress (NaCl) and with HDF cells with and without inflammation stress (TNF-alpha).

The effect of some of the strains on NHEK and HDF cells was investigated first by cell viability and confluency measurements. In addition to this, the effect of some of the strains on HDF cells was studied measuring the amount of synthesized type I pro-collagen and total-MMP-1 molecules produced.

### NHEK and HDF cells

Adult normal human epidermal keratinocytes (NHEKs) (Gibco™) were maintained in EpiLife® medium with 60 µM calcium (Gibco™), supplemented with Human Keratinocyte Growth Supplement (HKGS) (Gibco™) at 5 % CO₂ atmosphere, 37°C. For the experiments, the earliest possible passage was utilized.

Normal human dermal fibroblasts (HDFs) (Gibco™) were maintained in Medium 106 (Gibco™) supplemented with Low Serum Growth Supplement (LSGS) (Gibco™) at 5 % CO₂ atmosphere, 37°C. For the experiments, the earliest possible passage was utilized.

### Probiotic cell culture and test sample preparations

*Bifodobacterium animalis* spp. *lactis* strains B420 (DSM 32073) (commercially available from DuPont Nutrition Biosciences ApS), Bi-07 (commercially available from DuPont Nutrition Biosciences ApS) and BI-04 (commercially available from DuPont Nutrition Biosciences ApS) were cultured anaerobically at 37°C in *Bifidobacterum* medium No. 58 (DSMZ). For studies with live and lysed bacteria, and postbiotics, the bacteria were grown until exponential growth phase was reached. Bacteria cells and soluble metabolites were separated by centrifuging at 4°C, 5 min, 4000 rpm. Bacterial pellets were resuspended to either supplemented EpiLife® cell culture medium for keratinocyte studies, or to supplemented Medium 106 cell culture medium for dermal fibroblast studies. The bacterial cell densities were determined with optical density meter (BioPhotometer, Eppendorf) and flow cytometry (FACSCalibur, Becton Dickinson, San Jose, CA, USA). Bacterial cell lysates were prepared by shearing the cells with Precellys 24 bead beater (Bertin Technologies, Saint-Quentin-en-Yvelines Cedex, France) with grinding kit VK01 (Bertin Technologies) at 6800 rpm with 3 cycles of 30 seconds. The breakdown of the cells was confirmed by light microscopy. The bacterial cell lysates were sterile-filtered with 0.2 µm syringe units (Sartorius) before applying them to either keratinocytes or to dermal fibroblasts. The live and lysed bacterial cells were administered as 100 bacterial cells towards one keratinocyte or as 10 bacterial cells towards one dermal fibroblast. The soluble metabolites were diluted to 10 % (vol/vol) in supplemented EpiLife® cell culture medium or to 1 % (vol/vol) in supplemented Medium 106 and sterile-filtered with 0.2 µm syringe units (Sartorius) before applying them to cells.

Human transforming growth factor beta 1 (Sigma-Aldrich, Germany), used as positive control in HDF pro-collagen synthesis studies, was applied to HDF cells as 1 ng/ml concentration diluted in the supplemented Medium 106.

### Stress conditions

Hyper-osmotic stress for keratinocyte studies was induced by adding NaCl (J.T. Baker) to supplemented EpiLife® cell culture medium to reach 150 mM molar concentration, which corresponds about 570 mOsm/kg osmolality measured by osmometer (Ordior). Inlammation stress for the HDF studies was applied with human TNF-alpha (Life Technologies) diluted in supplemented Medium 106 to concentration of 20 ng/ml. The stresses were applied to the cells with the bacterial test solutions.

### MTT and confluency assays

MTT 3-(4,5-dimethylthiazol-2-yl)-2,5-diphenyltetrazolium bromide assay was used to evaluate the metabolic activity (viability) of the cells after the test sample incubation. Briefly, to assess the metabolic activity of NHEK and HDF cells, 10⁴ cells were plated in a well of 96-well plate (Nunc™ MicroWell™, Thermo Scientific) and after 24 h test samples in 0,1 ml volume were applied topically. After 24 h incubation with NHEK cells and after 72 h incubation with HDF cells, the test samples were removed, cells were washed twice with phosphate buffered saline (PBS) and treated with 0,5 mg/ml MTT (Sigma-Aldrich) -reagent solution (diluted in DMEM, Gibco) for 2 hours (NHEK cells) or 3 hours (HDF cells) at 37°C, 5 % CO₂ atmosphere. MTT-solution was collected, and replaced with 0,12ml of dimethyl sulfoxide (DMSO) (Sigma-Aldrich) and incubated in orbital shaker for 5 min protected from light. Volume of 0,09 ml of the supernatant was transferred to a 96-well plate and the absorbances of the samples were analysed with EnSight plate reader (Perkin Elmer, USA) measuring the 570 nm absorbance (reducing the background absorbance with 700 nm).

Confluency measurement was used to assess the cell morphology-related information by determining the area covered by cells. Briefly, to assess the cell confluency of NHEK cells, 10⁴ cells were plated in a well of 96-well plate (Nunc™ MicroWell™, Thermo Scientific) and after 24 h to let the cells to attach at 37°C, 5 % CO₂ atmosphere, test samples in 0,1 ml volume were applied topically. After 24 h incubation with NHEK cells at 37°C, 5 % CO₂ atmosphere, the test samples were removed, and cells were washed twice with phosphate buffered saline (PBS). Brightfield imaging with EnSight plate reader (Perkin Elmer, USA) and the accompanied software (Kaleido, Perkin Elmer, USA) was used to determine the cell confluency.

### ELISA assays

The amount of synthesized pro-collagen I in the HDF cell culture supernatant was analysed with a sandwich ELISA assay detecting natural and recombinant human pro-collagen I alpha 1 (Pro-Collagen I α1) (R&D Systems, USA) and the amount of produced matrix metalloproteinase 1 (MMP-1) in the HDF cell culture supernatant was analysed with a sandwich ELISA assay detecting natural and recombinant human active and pro-matrix metalloproteinase 1 (Total MMP-1) (R&D Systems, USA).

Briefly, 4x10⁴ HDF cells/well were plated in 24-well plates (Costar®, Corning) and incubated at 37°C, 5 % CO₂ atmosphere for 72h. Thereafter, test samples were applied to the cells in 1 ml volume, and incubation was continued for additional 72 h. After the incubation, cell culture supernatant was collected, and the cells were washed once with warm PBS and lyzed with CelLytic™ M reagent (Sigma-Aldrich) according to manufacturer's instructions. The protein concentration of the lysates was analysed with Pierce BCA Protein Assay Kit (Thermo Scientific) according to manufacturer's instructions.

Human pro-collagen I alpha 1 and human total MMP-1 were analysed from the cell culture supernatant samples according to the manufacturer's instructions. The results were normalized by the corresponding cell lysate protein concentration.

### Statistical analyses

The statistical significances between the treatments were determined using one-way ANOVA, considering P-values of 0.05 or less as significant. The analyses were calculated using GraphPad Prism version 8.4.2. showing mean ± SE values for all the results.

### Results

### Metabolic activity

The metabolic activity of the cells describes the general well-being of the cells. The MTT method measures the cellular metabolic activity of living cells, and the reduction potential of a cell or the availability of reducing compounds to drive cellular energetics. It is also utilized to measure viability of the cells. Absorbance values greater than the control indicate cell proliferation or higher metabolic activity, while lower values suggest cell death or inhibition of proliferation or lower metabolic activity. The decrease in MTT activity in cells is considered an indicator of cell redox activity. Senescent cells, or cells that are not able to divide normally accumulate in chronologically aged and photoaged skin; and may contribute to age-related skin changes and pathologies. Epidermal thinning, that is associated with aging skin, is, in part, caused by decreased proliferation and renewal capacity of basal keratinocytes and reduced epidermal stem cell number. Also, the dermal-epidermal junction (DEJ) that connects the epidermis to the underlying dermis as well as the dermis itself become thinner. Senescent cells lose their functionality and affect also surrounding cells by secreting various molecules, including pro-inflammatory chemokines, cytokines and various proteases. TNF-alpha, a pro-inflammatory cytokine can have various effects in different cells, from modulation of cell cycle and proliferation to inducing apoptosis and necrotic cell death. Long term TNF-alpha treatment has been seen to increase premature dermal fibroblast senescence by decreasing the cell proliferation potential.

One of the major functions of the skin is to provide a protection against dehydration and water loss. The skin of terrestrial animals, including humans, is exposed to an environment that varies in humidity from 0 to 100% relative humidity. Less than 100% relative humidity can produce significant dehydration or osmotic stress on the epidermis, which then must respond and protect the organism from water loss. Aged skin is drier than younger skin and has more rigid epidermis. Wrinkles are formed through the folding of epidermis along with bulk deformation of dermis, dehydrated rigid epidermis resists more to the folding stress, and hence shows less wavy surface, which means that dehydration generates wrinkles with larger amplitudes and periods. With an adequate amount of water in the SC, the skin maintains its intact barrier function, feels soft and flexible and looks smooth and healthy. Drier skin shows more wrinkles and deepers furrows with wider intervals.

### NHEK cell metabolic activity - No stress

The metabolic activity of NHEK cells was screened with MTT (3-(4,5-dimethylthiazol-2-yl)-2,5-diphenyltetrazolium bromide) assay after incubating the cells with test solutions for 24 hours. The number of viable cells present is reflected in the capability of NAD(P)H-dependent cellular oxidoreductase enzymes to reduce the MTT reagent to insoluble formazan, which can be measured by absorbance analysis. Live B420 and BI-04 bacteria were noticed to increase the metabolic activity (viability) of NHEK cells with statistical significancy (175.1% and 136.8%; p<0.0001 and p<0.05) compared to medium control having viability set as 100 % (Figure 1). Different forms of probiotic Bi-07: live, lysed and postbiotics, showed also beneficial effect, albeit less significant, compared to the medium control (132.5%, 119.8% and 108.4 %). In addition, lysed BI-04 cells had also somewhat higher metabolic activity compared to the medium control (108.7%).

This reflects the ability of the bacteria to maintain and even increase the metabolic activity/cell viability in the skin, which benefits the total skin well-being.

### NHEK cell metabolic activity - NaCl stress

The metabolic activity of NHEK cells under stress was screened with MTT (3-(4,5-dimethylthiazol-2-yl)-2,5-diphenyltetrazolium bromide) assay after incubating the cells with test solutions with hyperosmotic stress (induced by NaCl) for 24 hours. Hyperosmotic stress disturbs the cell osmotic balance and was used to model dehydration.

During hyperosmotic stress induced by NaCl, Bi-07 postbiotics (52.93%, p<0.0001), live B420 bacteria (55.32%, p<0,0001), B420 postbiotics (65.28%, p<0.0001) and BI-04 postbiotics (51.02%, p<0.0001) were able to increase the metabolic activity (viability) of NHEK cells compared to NaCl treated control cells (23.88%) (Figure 2). Although to a slightly lesser extent, also Bi-07 live (34.33%) and lysed (25.03%) as well as BI-04 live (27.75%) were all able to increase the metabolic activity compared to the NaCl treated cells.

Hyper-osmotic stress causes metabolic decrease in the cells and can cause cell senescence. Probiotic strains Bi-07, B420 and BI-04 were able to slow down the reduction of metabolic activity and/or cell viability caused by hyper-osmotic stress, which might refer to the ability to support the cell proliferation and viability, and equalize the osmotic difference in the skin during dehydration that can induce the signs of aging.

### HDF cell metabolic activity - No stress

The metabolic activity of HDF cells was screened with MTT (3-(4,5-dimethylthiazol-2-yl)-2,5-diphenyltetrazolium bromide) assay after incubating the cells with test solutions for 72 hours. Lysed B420 cells increased the metabolic activity (viability) the best (140.2%, p=0.0511) among the other samples compared to medium control having viability set as 100 % (Figure 3). In addition, Bi-07 lysed (110.4%) and postbiotics of all the three strains Bi-07 (136.5%), B420 (117.0%) and BI-04 (111.4%), although less significant, were also able to increase the HDF cell metabolic activity.

Adequate cell functioning is as important in the lower layers of the skin. Dermal fibroblast metabolic activity and viability is important e.g. to growth factor expression and the ECM protein metabolism, skin structural protein collagen being one of the important ones. Senescent fibroblasts are not able to function properly, which may lead to poor collagen synthesis and reduced structural support for the skin. The beneficial effects of the presented bacterial strains may reflect to the ability to support the skin cells proliferation and functionality.

### HDF cell metabolic activity - TNF-alpha stress

The metabolic activity of HDF cells was screened with MTT (3-(4,5-dimethylthiazol-2-yl)-2,5-diphenyltetrazolium bromide) assay after incubating the cells with test solutions with inflammation stress (induced by TNF-alpha) for 72 hours.

During inflammation stress induced by TNF-alpha, BI-04 postbiotics (189.1%, p<0.0001) showed the best beneficial effect on the cell metabolic activity (viability) of HDF cells compared to TNF-alpha treated control cells (135.42%) (Figure 4). In addition, Bi-07 (152.3%) and B420 (152.5%) postbiotics as well as lysed cells of B420 (145.3), they also increased the metabolic activity (viability) compared to the control.

Besides regulating immune and inflammatory responses, TNF-alpha also influences cell cycle, tissue remodelling and apoptosis. In the results, the influence of TNF-alpha in the cell proliferation may be reflected, as TNF-alpha itself also increased cell metabolic activity compared to the medium control normalized as 100 %. Nevertheless, the *Bifidobacterium* strains here were able to stimulate the metabolic activity of dermal fibroblast cells under the TNF-alpha stress, which may refer to the ability counteract the negative effects of the stress on the viability, and support the cell functionality for example under UV-stress which induces the TNF-alpha expression in the skin.

### Confluency

The visual inspection of the cells and measurement of confluency determines the percentual image area covered by cells, or the area from the culture dish that is covered by the cells. Increase in the confluency indicates either that the cell number has increased, or that cells are spreading more or adhere better to the substratum, or that the volume of the cells has increased. Epithelial sheet spreading is a fundamental cellular process that must be coordinated with cell division and differentiation to restore tissue integrity. Dividing keratinocytes are committed to differentiation, when the cell confluency with increased cellular density is high enough. Senescent cells present in aged skin cannot divide and for that reason it is of benefit that the confluency increases.

In hyperosmotic stress, cell volume is reduced, cell shrinkage occurs as a result of water efflux. In aged skin the expression of osmolyte transporters is reduced leading to reduced recovery of the cell volume from the osmotic stress. Hyper-osmotic stress has been also implicated in the proinflammatory signaling and thus in promotion of aging. It causes cessation of all major biosynthetic routes, including DNA synthesis and repair, transcription, protein translation and degradation, as well as mitochondrial function. As a result, cell cycle progression and cell proliferation are halted. There is a concomitant increase in oxidative stress and activation of apoptotic pathways, which might lead to reduction in cell number.

### NHEK cell confluency- No stress

After incubating the cells with test solutions for 24 hours, confluency, the area covered by the cells, was assessed with briedfield imaging and the accompanied software.

Live Bi-07 bacteria (47.09%, p=0.0001) as well as live B420 bacteria (43.83%, p=0.0365) had statistically significant increases in the NHEK cell confluency compared to medium control sample (38.45%) (Figure 5). Bi-07 lysed cells (43.57%) and BI-04 live cells (42.49%) also showed some increase in the cell confluency compared to the medium control.

The slight increase in the confluency might reflect the increase in cell amounts and adherence to the well substratum. In favourable conditions cells maintain, and even increase, the proliferation with a simultaneous balance in the cell volume regulation.

### NHEK cell confluency - NaCl stress

After 24h incubation in the precence of hyper-osmotic stress, which disturbs the cell osmotic balance, the area covered by the cells, confluency, was assessed with briedfield imaging and the accompanied software. The confluency analysis reveals interesting information especially under hyper-osmotic conditions, as it causes cell shrinkage when they try to balance the osmotic difference.

During the NaCl stress, several test compounds were able to restore the cell confluency compared to NaCl control sample (15.84%), among the others Bi-07 as live (24.60%, p=0.0038), lysed (25.90%, p=0.0007) and in postbiotic forms (32.76%, p<0.0001), B420 as live (24.94%, p=0.0024) and postbiotics (31.47%, p<0.0001) and BI-04 as postbiotics (25.90%, p=0.0007) (figure 6). All the other samples also had some increasing effect in the confluency compared to the hyper-osmotic stress control: B420 lysed (19.36%) and BI-04 as live (19.22%) and lysed (21.37%) (Figure 6).

The strains Bi-07, B420 and BI-04 were able to counteract the harmful effects of the hyper-osmotic condition, as they were able to attenuate the cell shrinkage and cell detaching that was observed as reduction in the cell confluency with NaCl stress control samples. This might refer to the ability of the three strains, Bi-07, B420 and BI-04 to support skin cells during osmotic stress and in dehydration that can cause skin aging.

### Pro-collagen synthesis

The pro-inflammatory cytokine tumor necrosis factor-alpha (TNF-α) inhibits collagen synthesis and induces matrix metalloproteinases (MMPs), and this has been associated with increase in collagen degration, which may disrupt skin integrity and cause skin aging. In aging skin, in the senescent cells the biosynthetic capability decreases leading to reduction in the amount of collagen. Increase in the pro-collagen is thus important to counterbalance the increase in the degradation of collagen and decrease in biosynthesis of pro-collagen observed in aged skin cells.

### Pro-collagen synthesis- No stress

The amount of synthesized pro-collagen I α1 in the HDF cell culture supernatant was analysed with ELISA-assay after incubating the cells with test solutions for 72 hours. Postbiotics of Bi-07 (1492 ng ml/mg protein, p<0.0001), B420 (1851 ng ml/mg protein, p<0.0001) and BI-04 (1799 ng ml/mg protein, p<0.0001) were all able to raise the pro-collagen I α1 production compared to medium control (451,8 ng ml⁻¹/mg protein) (Figure 7), in addition, Bi-07 (702,7 ng ml/mg protein, p=0.0075) and BI-04 lysed cells (666,2 ng ml/mg protein, p= 0.0275) were able to increase the production as well, although more modest level, as well as the B420 lysed cells (647,1 ng ml⁻¹/mg protein). The positive control TGF-beta1 for the collagen production was successfully noticed to increase pro-collagen I α1 synthesis (830,7 ng ml⁻¹/mg protein, p<0.0001) compared to the control.

All the strains were able to increase the pro-collagen synthesis better than the medium control, and postbiotics of Bi-07, B420 and BI-04 even doubled the amount of the positive control TGF-beta. This reflects to the ability of these strains to stimulate the dermal fibroblasts to increase pro-collagen production, which is especially valuable during skin aging, when the collagen production is dimished.

### Pro-collagen synthesis- TNF-α stress

The amount of synthesized pro-collagen I α1 in the HDF cell culture supernatant was analysed with ELISA-assay after incubating the cells with test solutions with inflammation stress (induced by TNF-alpha) for 72 hours. During the TNF-alpha stress, especially B420 and BI-04 postbiotics were able to have an effect increasing the type I pro-collagen synthesis (1218 ng ml/mg protein and 1227 ng ml/mg protein; p<0.0001 and p<0.0001), as well as Bi-07 postbiotics (620,6 ng ml/mg protein, p=0.0001) and BI-04 lysed cells (506,2 ng ml/mg protein, p=0.0420) and a bit lesser extend B420 lysed cells (472,1 ng ml/mg protein) (Figure 8). The positive control TGF-beta1 for the collagen production, was noticed to have a decreased level of pro-collagen I α1 synthesis under the inflammation stress (212,1 ng ml⁻¹/mg protein, p=0.0039).

The strains B420, BI-04 and Bi-07 were able to increase and preserve the pro-collagen production even under the inflammatory TNF-alpha stress, and the production remained even higher than with TGF-beta control, the function of which TNF-alpha is inhibiting, as the synthesized pro-collagen amount was even lower than with TNF-alpha control. These strains might be of benefit to support skin to maintain or even increase collagen synthesis under stress that otherwise could cause reduced synthesis leading diminished structural support in skin.

### MMP-1/pro-collagen ratio

The matrix metalloproteinases (MMPs) play an important role in skin aging. MMPs are induced in aged skin resulting degradation of dermal collagen and other structural proteins from the extracellular matrix thus resulting in wrinkles and sagging. It is important that the production of MMPs is coordinated with the production of collagen as increased expression of the MMPs without increase in collagen production leads to increase in aberrant collagen degration and this contributes to the wrinkling. Tumor necrosis factor (TNF)-α induces matrix metalloproteinases (MMPs), and this has been associated with increase in collagen degration, which may disrupt skin integrity and cause aging.

### MMP-1/pro-collagen ratio - No Stress

The ratio on produced total MMP-1 to synthesized pro-collagen I α1 by Human Dermal Fibroblast (HDF) cells to the culture supernatant was compared to medium control. The balance between collagen and MMP-1 is vital in the skin. Postbiotics of the three strains, Bi-07 (ratio 0.8471, p<0.0001), B420 (ratio 1.177, p=0.0004) and BI-04 (ratio 1.323, p=0.0010) were all able to maintain the total MMP-1/pro-collagen balance (ratio) (Figure 9), as the ratio was around the same level as the positive control TGF-beta1 for pro-collagen synthesis (ratio 0.9198, p<0.0001). Lysed Bi-07 cells were also able to have a lower total MMP-1/pro-collagen ratio (ratio 1.310, p= 0.0009) compared to the medium control sample (ratio 3.305), in addition, compared to the medium control, lysed B420 and BI-04 cells were also noticed to have a lower ratio (ratio 2.422 and 2.709).

All of the strains Bi-07, B420 and BI-04 were able preserve the total MMP-1/pro-collagen I - ratio under the medium control value, where the amount of produced MMP-1 was higher than the synthesized pro-collagen. When the balance between synthesized MMP-1 and produced pro-collagen maintains, the skin collagen metabolism is in homeostasis, it is not leading to excessive degradation of the structural protein collagen nor to abnormal accumulation of collagen.

All publications mentioned in the above specification are herein incorporated by reference. Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and biotechnology or related fields are intended to be within the scope of the following claims.

## Claims

1. Non-therapeutic use of a bacterium of the species *Bifidobacterium animalis* or a mixture thereof for preventing, reducing or treating skin aging in a subject.

2. The non-therapeutic use according to claim 1, wherein the bacterium of the species *Bifidobacterium animalis* is of the subspecies *Bifidobacterium animalis* ssp. *lactis.*

3. The non-therapeutic use according to claim 1 or 2, wherein the bacterium of the species *Bifidobacterium animalis* or the mixture thereof maintains metabolic activity/cell viability in the skin, increases the metabolic activity/cell viability in the skin or slows down the reduction of metabolic activity and/or cell viability in the skin.

4. The non-therapeutic use according to claims 1-3, wherein the bacterium of the species *Bifidobacterium animalis* or the mixture thereof increases or maintains production of type I pro-collagen or slows down the reduction of type I pro-collagen in the skin.

5. The non-therapeutic use according to claims 1-3, wherein the bacterium of the species *Bifidobacterium animalis* or the mixture thereof increases collagen production or maintains collagen metabolism or slows down the reduction of collagen metabolism in the skin.

6. The non-therapeutic use according to claims 1-3, wherein the bacterium of the species *Bifidobacterium animalis* or the mixture thereof maintains the total matrix metalloproteinase 1 (MMP-1)/pro-collagen balance.

7. The non-therapeutic use according to any one of the claims 1-6, wherein the bacterium of the species *Bifidobacterium animalis* is, or the mixture thereof comprises, strain 420 (B420).

8. The non-therapeutic use according to any one of the claims 1-6, wherein the bacterium of the species *Bifidobacterium animalis* is, or the mixture thereof comprises, strain Bi-07.

9. The non-therapeutic use according to any one of the claims 1-6, wherein the bacterium of the species *Bifidobacterium animalis* is, or the mixture thereof comprises, strain Bl-04.

10. The non-therapeutic use according to any one of the claims 1-6, wherein the bacterium of the species *Bifidobacterium animalis* is selected from the group consisting of strain 420 (B420), strain Bi-07, strain BI-04 and any mixture thereof.

11. The non-therapeutic use according to any one of the claims 1-10, wherein the bacterium of the species *Bifidobacterium animalis* or the mixture thereof are in a live form.

12. The non-therapeutic use according to any one of the claims 1-10, wherein the bacterium of the species *Bifidobacterium animalis* or the mixture thereof are in a lysed form.

13. The non-therapeutic use according to any one of the claims 1-10, wherein the bacterium of the species *Bifidobacterium animalis* or the mixture thereof are in a postbiotic form.

14. The non-therapeutic use according to any one of claims 1 to 13, wherein the bacterium of the species *Bifidobacterium animalis* or the mixture thereof is at least one component of a composition, a skin care composition, a dietary supplement, a nutritional supplement, a food product or a pharmaceutical acceptable composition.

15. The non-therapeutic use according to claim 14, wherein the composition, the skin care composition or the pharmaceutical acceptable composition are for topical application.

16. The non-therapeutic use according to claim 14, wherein the composition, the skin care composition, the dietary supplement, the nutritional supplement, the food product or the pharmaceutical acceptable composition are for oral administration.

17. Bacterium of the species *Bifidobacterium animalis* or a mixture thereof for use in prevention, reduction or treatment of skin aging in a subject.

18. The bacterium for use according to claim 17, wherein said bacterium of the species *Bifidobacterium animalis* is of the subspecies *Bifidobacterium animalis ssp. lactis.*

19. The bacterium for use according to claims 17 or 18, wherein the bacterium of the species *Bifidobacterium animalis* or the mixture thereof maintains metabolic activity/cell viability in the skin, increases the metabolic activity/cell viability in the skin or slows down the reduction of metabolic activity and/or cell viability in the skin.

20. The bacterium for use according to claims 17-19, wherein the bacterium of the species *Bifidobacterium animalis* or the mixture thereof increases or maintains production of type I pro-collagen or slows down the reduction of type I pro-collagen in the skin.

21. The bacterium for use according to claims 17-19, wherein the bacterium of the species *Bifidobacterium animalis* or the mixture thereof increases collagen production or maintains collagen metabolism or slows down the reduction of the collagen metabolism in the skin.

22. The bacterium for use according to claims 17-19, wherein the bacterium of the species *Bifidobacterium animalis* or the mixture thereof maintains total matrix metalloproteinase 1 (MMP-1)/pro-collagen balance.

23. The bacterium for use according to any one of the claims 17-22, wherein the bacterium of the species *Bifidobacterium animalis* is, or the mixture thereof comprises, strain 420 (B420).

24. The bacterium for use according to any one of the claims 17-22, wherein the bacterium of the species *Bifidobacterium animalis* is, or the mixture thereof comprises, strain Bi-07.

25. The bacterium for use according to any one of the claims 17-22, wherein the bacterium of the species *Bifidobacterium animalis* is, or the mixture thereof comprises, strain Bl-04.

26. The bacterium for use according to any one of the claims 17-22, wherein the bacterium of the species *Bifidobacterium animalis* is selected from the group consisting of strain 420 (B420), strain Bi-07, strain BI-04 and any mixture thereof.

27. The bacterium for use according to any one of the claims 17-22, wherein the bacterium of the species *Bifidobacterium animalis* or the mixture thereof are in a live form.

28. The bacterium for use according to any one of the claims 17-22, wherein the bacterium of the species *Bifidobacterium animalis* or the mixture thereof are in a lysed form.

29. The bacterium for use according to any one of the claims 17-22, wherein the bacterium of the species *Bifidobacterium animalis* or the mixture thereof are in a postbiotic form.

30. The bacterium for use according to any one of claims 17 to 29, wherein the bacterium of the species *Bifidobacterium animalis* or the mixture thereof is at least one component of a composition, a skin care composition, a dietary supplement, a nutritional supplement, a food product or a pharmaceutical acceptable composition.

31. The bacterium for use according to claim 30, wherein the composition, the skin care composition or the pharmaceutical acceptable composition are for topical application.

32. The bacterium for use according to claim 30, wherein the composition, the skin care composition, the dietary supplement, the nutritional supplement, the food product or the pharmaceutical acceptable composition are for oral administration.
